# EUROPEAN PATENT SPECIFICATION

(11) **EP 2 653 091 B1**
(45) Date of publication and mention of the grant of the patent: **10.06.2015**
(21) Application number: 12765092.7
(22) Date of filing: 01.03.2012
(51) Int. Cl.: A61B 1/00, A61B 1/04, A61B 1/06, G02B 23/24

(54) **SCANNING ENDOSCOPE DEVICE**
ABTASTENDOSKOP
ENDOSCOPE DE BALAYAGE

(30) Priority: 31.03.2011 JP 2011080634
(43) Date of publication of application: 23.10.2013
(73) Proprietor: Olympus Medical Systems Corp., Tokyo 151-0072 (JP)
(72) Inventor: SHIMADA, Tomoko, Tokyo 151-0072 (JP); YOSHINO, Masahiro, Tokyo 151-0072 (JP); IGARASHI, Makoto, Tokyo 151-0072 (JP)
(74) Representative: Gunzelmann, Rainer
(86) International application number: PCT/JP2012/055186
(87) International publication number: WO 2012/132750

(56) References cited:
- WO-A1-2006/080076
- JP-A- H05 504 845
- JP-A- 2011 036 592
- JP-A- 2011 504 782
- US-A1- 2009 137 893

## Description

### {Technical Field}

The present invention relates to scanning endoscope devices.

### {Background Art}

Conventional scanning endoscope devices that obtain two images from different viewpoints (parallax images) by irradiating mutually displaced points on an observation target with two light beams while two-dimensionally scanning the light beams are known (e.g., see Patent Document 1). It is possible to stereoscopically view the observation target by using such parallax images.

### {Citation List}

### {Patent Literature}

### {PTL 1}

Specification of United States Patent Application Publication No. 2009/0137893

US 2009/137893 discloses in combination: a scanning endoscope device that obtains a parallax image, comprising a first core portion that radiates an illuminating light beam for a first viewpoint toward a subject, the illuminating light beam having a first optical characteristic; a second core portion that is provided parallel to the first core portion and that radiates an illuminating light beam for a second viewpoint toward the subject, the illuminating light beam having a second optical characteristic different from the first optical characteristic; driving units that two-dimensionally scan the illuminating light beams radiated from the first core portion and the illuminating light beam radiated from the second core portion, each, by causing vibration of the distal-end portions of the first core portion and the second core portion together; a light receiving unit that receives return light beams, returned from the subject, of the illuminating light beam radiated from the first core portion and the illuminating light beam radiated from the second core portion; a light splitting unit that splits the return light beams received by the light receiving unit into a return light beam having the first optical characteristic and a return light beam having the second optical characteristic; and an image generator.

### {Summary of Invention}

### {Technical Problem}

However, in the case of Patent Document 1, actuators for scanning light beams are provided, one for each light beam, at the distal-end portion of an inserted portion. This results in a disadvantage that the outer diameter of the inserted portion becomes large.

The present invention has been made in view of the situation described above, and it is an object thereof to provide a scanning endoscope device in which the diameter of an inserted portion can be made small while making it possible to obtain images from multiple viewpoints, enabling stereoscopic observation.

### {Solution to Problem}

In order to achieve the above objective, the present invention provides a scanning endoscope device according to claim 1.

According to the present invention, the driving unit two-dimensionally scans the illuminating light beams radiated from the two core portions of the optical fiber component to mutually displaced points on a subject. Thus, the image generating unit can generate parallax images consisting of images of multiple scanning areas displaced from each other, i.e., images observed from multiple viewpoints.

In this case, by using the common driving unit to cause vibration of the multiple core portions, the area occupied by the driving unit with respect to the radial direction of the inserted portion is kept small. Thus, the diameter of the inserted portion can be made small.

In the above invention, the illuminating light beams radiated from the at least two core portions may have mutually different wavelengths, and the detecting unit may be constructed to include a wavelength splitting mechanism that splits the return light beams received by the light receiving unit on the basis of wavelengths and at least two light detectors that detect the return light beams having individual wavelengths split by the wavelength splitting mechanism.

With this construction, the image generating unit generates images of the individual scanning areas from information about the return light beams having the individual wavelengths, detected by the individual light detectors. Thus, images of the subject are obtained by using light beams having different wavelengths, making it possible to observe characteristics of the subject in relation to light beams having the individual wavelengths from the individual images.

In the above invention, the illuminating light beams radiated from the at least two core portions may have mutually different polarization directions, and the detecting unit may be constructed to include a polarized-light splitting mechanism that splits the return light beams received by the light receiving unit on the basis of polarization directions and at least two light detectors that detect the return light beams having individual polarization directions split by the polarized-light splitting mechanism.

With this construction, the image generating unit generates images of the individual scanning areas from information about the return light beams having the individual polarization directions, detected by the individual light detectors. Thus, images of the subject are obtained by using light beams having different polarization directions, making it possible to observe characteristics of the subject in relation to light beams having the individual polarization directions from the individual images. Furthermore, it is possible to use light beams in the same wavelength range as the illuminating light beams radiated from the individual core portions.

In the above construction including the wavelength splitting mechanism or the polarized-light splitting mechanism, an illuminating unit that makes the illuminating light beams incident on the at least two core portions from the proximal-end side of the core portions may be provided, and the illuminating unit may simultaneously make the illuminating light beams incident on the at least two core portions.

With this construction, it is possible to simultaneously obtain multiple images from different viewpoints.

In the above invention, an illuminating unit that makes the illuminating light beams incident on the at least two core portions from the proximal-end side of the core portions may be provided, and the illuminating unit may make the illuminating light beams incident on the at least two core portions in a time-division multiplexing fashion.

With this construction, it is possible to obtain multiple images from multiple viewpoints substantially simultaneously. Furthermore, it is possible to alleviate the effects of the illuminating light beams on the scanning areas by shortening the cumulative periods during which the scanning areas are irradiated with the illuminating light beams.

In the above invention, an optical component that is provided on the distal-end side of the optical fiber component to cause condensing of the illuminating light beams may be provided.

With this construction, it is possible to enhance the image resolution by reducing the spot diameter of the illuminating light beams radiated toward the subject.

In the above invention, a control unit that synchronizes the driving unit and the image generating unit with each other so that images of the return light beams are formed in accordance with vibration of the optical fiber component may be provided.

### {Advantageous Effects of Invention}

By means of the present invention, an advantage is afforded in that the diameter of an inserted portion can be made small while making it possible to obtain images from multiple viewpoints, enabling stereoscopic observation.

### {Brief Description of Drawings}

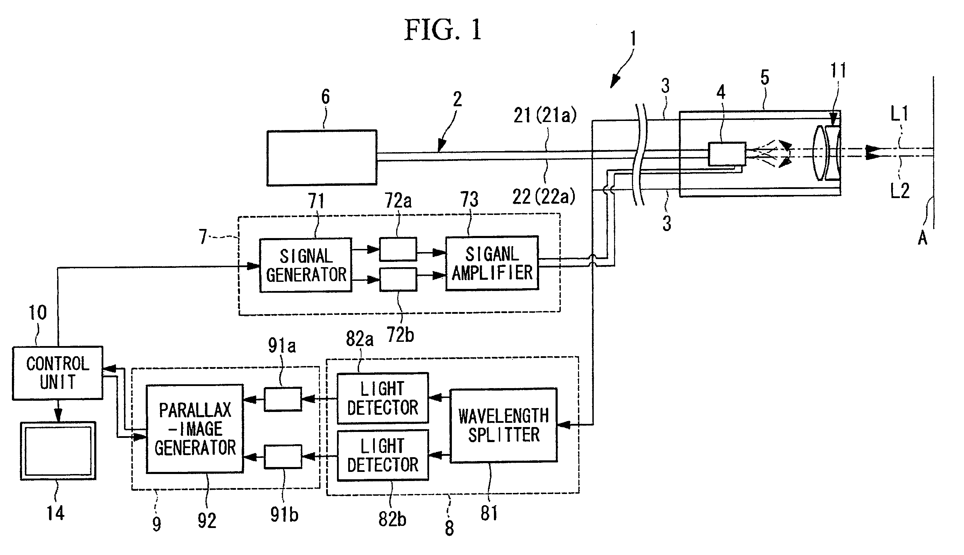

{Fig. 1}
   Fig. 1 is an overall construction diagram of a scanning endoscope device according to an embodiment of the present invention.
{Fig. 2}
   Fig. 2 is an enlarged view of the distal-end portions of light-emitting fibers in Fig. 1.
{Fig. 3}
   Fig. 3 is an illustration showing the distal-end face of an inserted portion in Fig. 1.
{Fig. 4}
   Fig. 4 is a diagram showing two scanning areas where illuminating light beams are scanned by the scanning endoscope device in Fig. 1.
{Fig. 5}
   Fig. 5 is an illustration showing a modification of the light-emitting fibers in Fig. 1.
{Fig. 6}
   Fig. 6 is an illustration showing a construction in which GRIN lenses are provided at the distal-end faces of the light-emitting fibers in Fig. 2.
{Fig. 7}
   Fig. 7 is an illustration showing a construction in which ball lenses are provided at the distal-end faces of the light-emitting fibers in Fig. 2.

### {Description of Embodiments}

A scanning endoscope device 1 according to an embodiment of the present invention will be described below with reference to the drawings.

The scanning endoscope device 1 according to this embodiment obtains parallax images that enable stereoscopic viewing by the parallel method. As shown in Fig. 1, the scanning endoscope device 1 includes an inserted portion 5 having light-emitting fibers (optical fiber component) 2 that emit illuminating light beams L1 and L2, light-receiving fibers 3, and an actuator (driving unit) 4 that causes vibration of the distal-end portions of the light-emitting fibers 2; an illumination unit 6 that supplies the illuminating light beams L1 and L2 to the light-emitting fibers 2; a driving unit 7 that drives the actuator 4; a detection unit (detecting unit) 8 that performs photoelectric conversion of return light beams of the illuminating light beams L1 and L2 received by the light-receiving fibers 3; an image generation unit 9 that generates parallax images based on signals from the detection unit 8; and a control unit 10 that controls the operation of the illumination unit 6 and the driving unit 7 and that outputs the parallax images generated by the image generation unit 9 to a monitor 14.

The light-emitting fibers 2 and the light-receiving fibers 3 are disposed along the lengthwise direction inside the inserted portion 5. At the distal end of the light-emitting fibers 2, an illumination optical system 11 is provided.

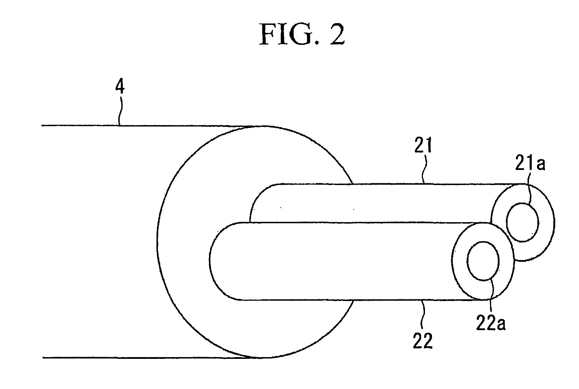

As shown in Fig. 2, the light-emitting fibers 2 include two optical fibers 21 and 22 that are joined together at least at their distal-end portions. The optical fibers 21 and 22 are single-mode fibers having cores (core portions) 21a and 22a, respectively. A first illuminating light beam L1 emitted from one core 21a and a second illuminating light beam L2 emitted from the other core 22a are condensed by the illumination optical system 11 and irradiate an observation surface A.

Here, as will be described later, the wavelength of the first illuminating light beam L1 and the wavelength of the second illuminating light beam L2 mutually differ. Therefore, because of aberrations that arise when these illuminating light beams L1 and L2 pass through the illumination optical system 11, the illuminating light beams L1 and L2 irradiate points on the observation surface A that are displaced in a direction crossing the optical axes.

At this time, preferably, the displacement d between the two illuminating light beams L1 and L2 is, for example, greater than or equal to about 80 µm and less than or equal to about 500 µm. Considering the diameter of each of the optical fibers 21 and 22, it is difficult to make the displacement d between the irradiated points less than 80 µm. On the other hand, a displacement d between the irradiated points greater than 500 µm is undesirable since the diameter of the inserted portion 5 becomes large. The displacement d between the irradiated points can also be designed by adjusting the distance between the two cores 21a and 22a, the emitting directions of the illuminating light beams L1 and L2 from the individual cores 21a and 22a, etc.

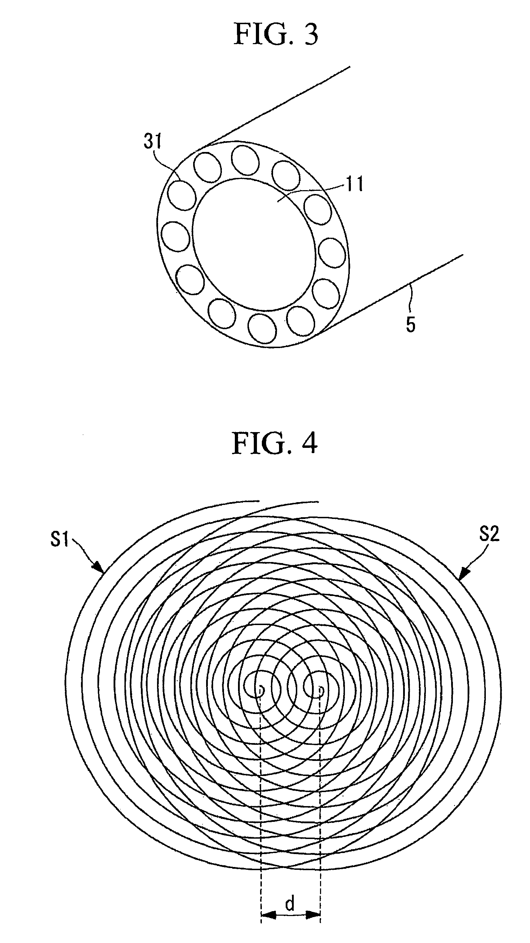

The light-receiving fibers 3 commonly receive return light beams of the two illuminating light beams L1 and L2 with light receiving faces (light receiving unit) 31 formed of the distal-end faces thereof and guide the received return light beams to the detection unit 8. Here, as shown in Fig. 3, multiple (12 in the example shown in the figure) light-receiving fibers 3 are provided, and the light receiving faces 31 are arranged to surround the illumination optical system 11 in the circumferential direction on the distal-end face of the inserted portion 5. This serves to increase the intensity of the return light received from the observation surface A.

The actuator 4 is, for example, an electromagnetic or piezoelectric actuator. When driving voltages (described later) are applied from the driving unit 7, the actuator 4 causes the distal-end portions of the light-emitting fibers 2 to vibrate in the directions of two axes (X direction and Y direction) crossing the lengthwise direction of the light-emitting fibers 2. Thus, the two illuminating light beams L1 and L2 are simultaneously scanned two-dimensionally on the observation surface A. There is no particular limitation about the scanning method, and spiral scanning, raster scanning, etc. can be used.

Here, since the distal-end portions of the two optical fibers 21 and 22 are joined together, the scanning trajectories of the two illuminating light beams L1 and L2 have the same shape, as shown in Fig. 4. Furthermore, scanning areas S1 and S2 (areas scanned by spiral scanning in the example shown in the figure) on the observation surface A scanned with the two illuminating light beams L1 and L2 are displaced by the displacement d between the points irradiated with the two illuminating light beams L1 and L2.

The illumination unit 6 is constructed to make the first illuminating light beam L1 having a first wavelength incident on one core 21a and to make the second illuminating light beam L2 having a second wavelength, which differs from the first wavelength, incident on the other core 22a. The first illuminating light beam L1 and the second illuminating light beam L2 are single-wavelength continuous-wave light. The first wavelength and the second wavelength are, for example, 532 nm and 440 nm. The illumination unit 6 is constructed of, for example, two light sources that individually emit the first illuminating light beam L1 and the second illuminating light beam L2. As the light sources, single-wavelength solid-state lasers, which have superior light guiding efficiency, are preferable.

The driving unit 7 includes a signal generator 71 that generates driving signals for driving the actuator 4 in the form of digital signals, D/A converters 72a and 72b that convert the driving signals generated by the signal generator 71 into analog signals, and a signal amplifier 73 that amplifies outputs of the D/A converters 72a and 72b.

The signal generator 71 generates two driving signals for vibrating the light-emitting fibers 2 in the X direction and Y direction and inputs the two driving signals to the separate D/A converters 72a and 72b. The signal amplifier 73 amplifies the analog signals generated by the D/A converters 72a and 72b, i.e., driving voltages, to an amplitude suitable for driving the actuator 4 and outputs the amplified driving voltages to the actuator 4.

The detecting unit 8 includes a wavelength splitter (wavelength splitting mechanism) 81 that splits return light beams guided by the individual light-receiving fibers 3 on the basis of their wavelengths and two light detectors 82a and 82b that detect the individual return light beams split by the wavelength splitter 81 and that performs photoelectric conversion.

The wavelength splitter 81 extracts a return light beam having the first wavelength and a return light beam having the second wavelength among the input return light beams and outputs these return light beams to the separate light detectors 82a and 82b.

The light detectors 82a and 82b are, for example, photodiodes or photomultiplier tubes. The light detectors 82a and 82b output photocurrents having magnitudes corresponding to the intensities of the detected return light beams to A/D converters 91a and 91b, respectively.

The image generation unit 9 includes two A/D converters 91a and 91b that convert the photocurrents output from the individual light detectors 82a and 82b into digital signals and a parallax-image generator 92 that generates two-dimensional images from the digital signals generated by the individual A/D converters 91a and 91b.

The parallax-image generator 92 generates two two-dimensional images based on the digital signals received from the individual A/D converters 91a and 91b and information about the scanning positions of the illuminating light beams L1 and L2 (described later) received from the control unit 10. Here, the two two-dimensional images are an image generated from the return light beam from the scanning area S1 scanned with the first illuminating light beam L1 and an image generated from the return light beam from the scanning area S2 scanned with the second illuminating light beam L2. That is, the two two-dimensional images are images whose viewpoints are shifted in parallel by an amount corresponding to the displacement d between the points irradiated with the two illuminating light beams L1 and L2. It is possible to construct a parallax image from these two two-dimensional images.

The control unit 10 outputs specification signals giving the specifications of the driving signals, e.g., the frequency, amplitude, etc., to the signal generator 71 and outputs information about the specification signals, i.e., information including the scanning positions of the illuminating light beams L1 and L2, to the parallax-image generator 92.

Furthermore, the control unit 10 reconstructs an image suitable for stereoscopic observation from the two two-dimensional images received from the parallax-image generator 92 and displays the reconstructed image on the monitor 14. This enables an operator to stereoscopically observe an image of the observation surface A generated by the scanning endoscope device 1.

In this case, according to this embodiment, even though the construction is such that parallax images are obtained by using the two illuminating light beams L1 and L2, the single actuator 4 suffices to scan the two illuminating light beams L1 and L2, so that an advantage is afforded in that the diameter of the inserted portion 5 can be made small. Furthermore, since images of the observation surface A are obtained by using the illuminating light beams L1 and L2 having different wavelengths, it becomes possible to perform simultaneous observation using light beams in different wavelength ranges. For example, by modifying the first illuminating light beam L1 to an excitation light beam for a fluorescent pigment (e.g., a near-infrared light beam), modifying the second excitation light beam L2 to a white light beam in which light beams from three solid-state lasers for RGB are combined, and suitably modifying the wavelengths of the return light beams split by the wavelength splitter 81, it becomes possible to simultaneously observe a fluorescence image and a white-light image.

Although the illuminating light beams L1 and L2 radiated from the individual cores 21a and 22a have mutually different wavelengths in this embodiment, alternatively, the illuminating light beams L1 and L2 may have mutually different polarization directions. In this case, the illumination unit 6 includes, for example, two polarizers that extract light beams having different polarization directions and that output the light beams to the individual cores 21a and 22a. Furthermore, a polarized-light splitter (not shown, polarized-light splitting mechanism) that extracts light beams having the individual polarization directions is provided between the observation surface A and the light receiving faces 31.

Also with this construction, it is possible to separately detect return light beams from the individual scanning areas S1 and S2 and to separately generate images of the individual scanning areas S1 and S2. Furthermore, it becomes possible to use light beams having the same wavelength as the first illuminating light beam L1 and the second illuminating light beam L2.

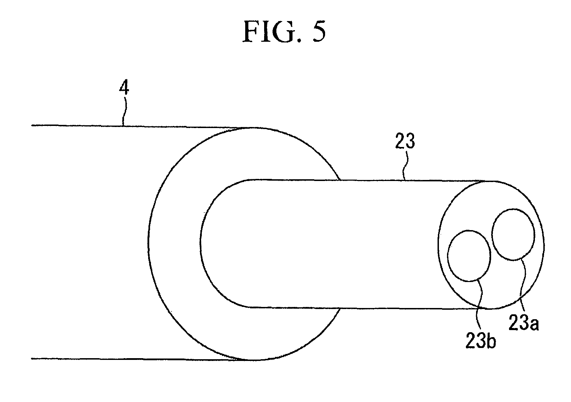

Furthermore, although the light-emitting fibers 2 include the two optical fibers 21 and 22 having a single core in this embodiment, alternatively, the light-emitting fiber 2 may consist of a single optical fiber 23 having two cores 23a and 23b, as shown in Fig. 5.

Also with this construction, it is possible to obtain parallax images by two-dimensionally scanning two illuminating light beams irradiating points that are displaced in a direction crossing the optical axes, simultaneously by means of the single actuator 4.

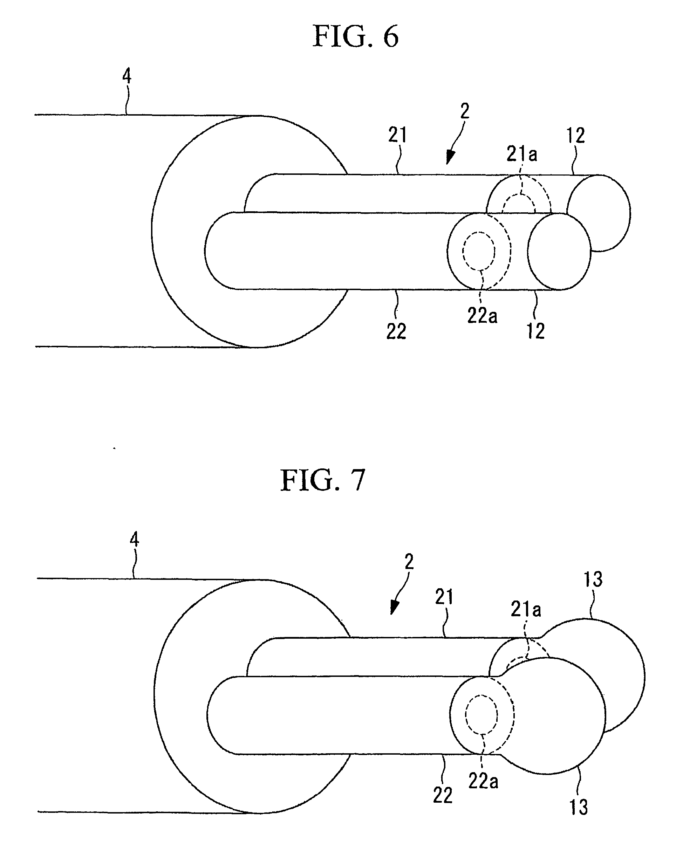

Furthermore, in this embodiment, optical components that condense the illuminating light beams L1 and L2 emitted from the individual cores 21a and 22a into collimated light beams or into smaller spot diameters may be joined at the distal-end faces of the two optical fibers 21 and 22. As the optical components, for example, GRIN (gradient index) lenses 12, shown in Fig. 6, or ball lenses 13, shown in Fig. 7, are used. This serves to improve the resolution of the parallax images. In the case where optical components are provided as described above, the illumination optical system 11 may be omitted.

Furthermore, although continuous light beams are used as the illuminating light beams L1 and L2 in this embodiment, alternatively, pulsed light beams may be used.

With this construction, since the cumulative irradiation periods of the observation surface A with the illuminating light beams L1 and L2 become shorter, the effects exerted on the observation surface A by the illuminating light beams L1 and L2 can be alleviated. For example, in the case of fluorescence observation, fading of the fluorescent pigment can be prevented. Furthermore, in the case where the observation surface A is irradiated with the first illuminating light beam L1 and the second illuminating light beam L2 in a time-division multiplexing fashion, it is possible to perform time-resolved measurement of the behavior of biological molecules, etc. on the observation surface A.

In the case where pulsed light beams are used as the illuminating light beams L1 and L2, the illumination unit 6 may be constructed to make the two illuminating light beams L1 and L2 incident on the individual cores 21a and 22a at pulse timings shifted from each other, and the detection unit 8 may be constructed to detect return light beams in synchronization with the pulse timings. In this construction, the wavelengths of the illuminating light beams L1 and L2 may be either the same or different. The latter case is suitable for fluorescence imaging using two different fluorescent pigments.

Furthermore, although the light-emitting fibers 2 include the two cores 21a and 22a in this embodiment, alternatively, the light-emitting fibers 2 may include three or more cores. For example, even in the case where the distal-end portions of three or more optical fibers having a single core are joined together, the single actuator 4 suffices to scan illuminating light beams from all the cores. Therefore, it is possible to obtain images of the observation surface A by using three or more illuminating light beams while making the diameter of the inserted portion 5 small.

### {Reference Signs List}

- 1: Scanning endoscope device
- 2: Light-emitting fibers
- 3: Light-receiving fibers
- 4: Actuator (driving unit)
- 5: Inserted portion
- 6: Illumination unit (illuminating unit)
- 7: Driving unit
- 8: Detection unit (detecting unit)
- 9: Image generation unit (image generating unit)
- 10: Control unit
- 11: Illumination optical system
- 12: GRIN lenses (optical components)
- 13: Ball lenses (optical components)
- 14: Monitor
- 21, 22, 23: Optical fibers (optical fiber component)
- 21a, 22a, 23a, 23b: Cores (core portions)
- 31: Light receiving faces (light receiving unit)
- 71: Signal generator
- 72a, 72b: D/A converters
- 73: Signal amplifier
- 81: Wavelength splitter (wavelength splitting mechanism)
- 82a, 82b: Light detectors
- 91a, 91b: A/D converters
- 92: Parallax-image generator
- A: Observation surface
- L1: First illuminating light beam
- L2: Second illuminating light beam

## Claims

1. A scanning endoscope device (1) that obtains a parallax image, comprising:
a first core portion (21a) that radiates an illuminating light beam (L1) for a first viewpoint toward a subject (A), the illuminating light beam (L1) having a first optical characteristic;
a second core portion (21b) that is provided parallel to the first core portion (21a) and that radiates an illuminating light beam (L2) for a second viewpoint toward the subject (A) simultaneously with the radiation by the first core portion (21a), the illuminating light beam (L2) having a second optical characteristic different from the first optical characteristic;
one driving unit (4, 7) that is provided in common with the first core portion (21a) and the second core portion (21b) and that two-dimensionally scans the illuminating light beam (L1) radiated from the first core portion (21a) and the illuminating light beam (L2) radiated from the second core portion (21b) by causing vibration of the distal-end portions of the first core portion (21a) and the second core portion (21b) together;
a light receiving unit (31) that receives return light beams, returned from the subject (A), of the illuminating light beam (L1) radiated from the first core portion (21a) and the illuminating light beam (L2) radiated from the second core portion (21b);
a light splitting unit (81) that splits the return light beams received by the light receiving unit (31) into a return light beam having the first optical characteristic and a return light beam having the second optical characteristic;
a first light detecting unit (82a) that photoelectrically converts the return light beam split by the light splitting unit (81) and having the first optical characteristic to output a first captured image signal for the first viewpoint;
a second light detecting unit (82b) that photoelectrically converts the return light beam split by the light splitting unit (81) and having the second optical characteristic to output a second captured image signal for the second viewpoint; and
an image generating unit (9) that generates a first image for the first viewpoint based on the first captured image signal output from the first light detecting unit (81a) and that generates a second image for the second viewpoint based on the second captured image signal output from the second light detecting unit (81b).

2. A scanning endoscope device (1) according to Claim 1,
wherein the first optical characteristic is a first wavelength range, and the second optical characteristic is a second wavelength range, which differs from the first wavelength range.

3. A scanning endoscope device (1) according to Claim 1,
wherein the first optical characteristic is a first polarization direction, and the second optical characteristic is a second polarization direction, which differs from the first polarization direction.

4. A scanning endoscope device (1) according to Claim 1, comprising a light-source controller (10) that controls the illuminating light beam (L1) radiated from the first core portion (21a) and having the first optical characteristic and the illuminating light beam (L2) radiated from the second core portion (21b) and having the second optical characteristic so that the subject (A) is irradiated simultaneously.

5. A scanning endoscope device (1) according to Claim 1, comprising a light-source controller (10) that controls the illuminating lightbeam (L1) radiated from the first core portion (21a) and having the first optical characteristic and the illuminating light beam (L2) radiated from the second core portion (21b) and having the second optical characteristic so that the subject (A) is irradiated in a time-division multiplexing fashion.

6. A scanning endoscope device (1) according to Claim 1, comprising an optical component (12, 13) that is provided on the distal-end side of the first core portion (21a) and the second core portion (21b) to cause condensing of the illuminating light beams (L1, L2).

7. A scanning endoscope device (1) according to Claim 1, comprising a control unit (10) that synchronizes the one driving unit (4, 7) and the image generating unit (9) with each other so that images of the return light beams are formed in accordance with vibration of the first core portion (21a) and the second core portion (21b) caused by the one driving unit (4, 7).

8. A scanning endoscope device (1) according to Claim 1, the one driving unit (4, 7) vibrates the distal-end portions of the first core portion (21a) and the second core portion (21b) together in such a way that scanning trajectories (S1, S2) of the illuminating light beam (L1) radiated from the first core portion (21a) and the illuminating light beam (L2) radiated from the second core portion (21b) are displaced each other by a predetermined distance (d) and have the same shape as each other.

9. A scanning endoscope device (1) according to Claim 8, wherein the light receiving unit (31) receives the return light beams at a predetermined position, and
wherein the image generating unit (9) generates images whose viewpoints are shifted each other in parallel by an amount corresponding to a predetermined distance as the first and the second images, on the basis of information including scanning positions of the illuminating light beam (L1) radiated from the first core portion (21a) and the illuminating light beam (L2) radiated from the second core portion (21b).

## Patentansprüche

1. Abtast-Endoskopvorrichtung (1), die ein Parallaxbild erlangt und aufweist:
ein erstes Kernteil (21a), das einen Beleuchtungslichtstrahl (L1) für einen ersten Ansichtspunkt zu einem Subjekt (A) strahlt, wobei der Beleuchtungslichtstrahl (L1) eine erste optische Charakteristik aufweist;
ein zweites Kernteil (21b), das parallel zu dem ersten Kernteil (21) bereitgestellt ist und das einen Beleuchtungslichtstrahl (L2) für einen zweiten Ansichtspunkt zu dem Subjekt (A) simultan mit der Bestrahlung durch das erste Kernteil (21a) strahlt, wobei der Beleuchtungslichtstrahl (L2) eine zweite optische Charakteristik aufweist, die sich von der ersten optischen Charakteristik unterscheidet;
eine Antriebseinheit (4, 7), die gemeinsam mit dem ersten Kernteil (21a) und dem zweiten Kernteil (21b) bereitgestellt ist und die zweidimensional den von dem ersten Kernteil (21a) abgestrahlten Beleuchtungslichtstrahl (L1) und den von dem zweiten Kernteil (21b) abgestrahlten Beleuchtungslichtstrahl (L2) abtastet durch Verursachen gemeinsamer Vibration der Distalendteile des ersten Kernteils (21a) und des zweiten Kernteils (21b);
eine Lichtempfangseinheit (31), die zurückkehrende Lichtstrahlen empfängt, die zurückkehren von dem Subjekt (A), vom dem durch das erste Kernteil (21a) abgestrahlten Beleuchtungslichstrahl (L1) und dem von dem zweiten Kernteil (21b) abgestrahlten Beleuchtungslichstrahl (L2);
eine Lichtteilungseinheit (81), die die von der Lichtempfangseinheit (31) zurückkehrenden Lichtstrahlen in einen Rückkehrlichtstrahl mit der ersten optischen Charakteristik und einen Rückkehrlichtstrahl mit der zweiten optischen Charakteristik teilt;
eine erste Lichterfassungseinheit (82a), die den Rückkehrlichtstrahl photoelektrisch wandelt, der durch die Lichtteilungseinheit (81) geteilt wurde, und die erste optische Charakteristik aufweist, um ein erstes abgefangenes Bildsignal für den ersten Ansichtspunkt auszugeben;
eine zweite Lichterfassungseinheit (82b), die den Rückkehrlichtstrahl photoelektrisch wandelt, der durch die Lichtteilungseinheit (81) geteilt wurde, und die zweite optische Charakteristik aufweist, um ein zweites abgefangenes Bildsignal für den zweiten Ansichtspunkt auszugeben; und
eine Bilderzeugungseinheit (9), die ein erstes Bild für den ersten Ansichtspunkt basierend auf dem von der ersten Lichterfassungseinheit (81a) ausgegebenen ersten abgefangenen Bildsignal erzeugt und die ein zweites Bild für den zweiten Ansichtspunkt basierend auf dem von der zweiten Lichterfassungseinheit (81b) ausgegebenen zweiten abgefangenen Bildsignal erzeugt.

2. Abtast-Endoskopvorrichtung (1) nach Anspruch 1,
bei der die erste optische Charakteristik ein erster Wellenlängenbereich ist, und die zweite optische Charakteristik ein zweiter Wellenlängenbereich ist, der sich von dem ersten Wellenlängenbereich unterscheidet.

3. Abtast-Endoskopvorrichtung (1) nach Anspruch 1,
bei der die erste optische Charakteristik eine erste Polarisationsrichtung ist, und die zweite optische Charakteristik eine zweite Polarisationsrichtung ist, die sich von der ersten Polarisationsrichtung unterscheidet.

4. Abtast-Endoskopvorrichtung (1) nach Anspruch 1 aufweisend eine Lichtquellensteuerung (10), die den Beleuchtungslichtstrahl (L1), der von dem ersten Kernteil (21a) abgestrahlt wurde und die erste optische Charakteristik aufweist, und den zweiten Beleuchtungslichtstrahl (L2), der von dem zweiten Kernteil (21b) abgestrahlt wurde und die zweite optische Charakteristik aufweist, steuert, so dass das Subjekt (A) simultan bestrahlt wird.

5. Abtast-Endoskopvorrichtung (1) nach Anspruch aufweisend eine Lichtquellensteuerung (10), die den Beleuchtungslichtstrahl (L1), der von dem ersten Kernteil (21a) abgestrahlt wurde und die erste optische Charakteristik aufweist, und den zweiten Beleuchtungslichtstrahl (L2), der von dem zweiten Kernteil (21b) abgestrahlt wurde und die zweite optische Charakteristik aufweist, steuert, so dass das Subjekt (A) auf eine zeitmultiplexweise bestrahlt wird.

6. Abtast-Endoskopvorrichtung (1) nach Anspruch 1 aufweisend eine optische Komponente (12, 13), die auf der Distalendseite des ersten Kernteils (21a) und des zweiten Kernteils (21b) bereitgestellt ist, um eine Kondensierung der Beleuchtungslichtstrahlen (L1, L2) zu verursachen.

7. Abtast-Endoskopvorrichtung (1) nach Anspruch 1 aufweisend eine Steuerungseinheit (10), die die eine Antriebseinheit (4, 7) und die Bilderzeugungseinheit (9) miteinander synchronisiert, so dass Bilder der Rückkehrlichtstrahlen gebildet werden in Übereinstimmung mit Vibration des ersten Kernteils (21a) und des zweiten Kernteils (21b) verursacht durch die eine Antriebseinheit (4, 7).

8. Abtast-Endoskopvorrichtung (1) nach Anspruch 1, bei der die eine Antriebseinheit (4, 7) die Distalendteile des ersten Kernteils (21a) und des zweiten Kernteils (21b) zusammen auf eine solche Art vibriert, dass Abtasttrajektorien (S1, S2) des Beleuchtungslichtstrahls (L1), der von dem ersten Kernteil (21a) abgestrahlt wurde, und dem Beleuchtungslichtstrahl (L2), der von dem zweiten Kernteil (21b) abgestrahlt wurde voneinander mit einem vorbestimmten Abstand (d) versetzt sind und die gleiche gegenseitige Form aufweisen.

9. Abtast-Endoskopvorrichtung (1) nach Anspruch 8, bei der die Lichtempfangseinheit (31) die Rückkehrlichtstrahlen an einer vorbestimmten Position empfängt und
bei der die Bilderzeugungseinheit (9) Bilder erzeugt, deren Ansichtspunkte voneinander parallel mit einem Betrag verschoben sind, der einem vorbestimmten Abstand wie die ersten und zweiten Bildern entspricht, auf der Basis von Information enthaltend Abtastpositionen des von dem ersten Kernteil (21a) abgestrahlten Beleuchtungslichtstrahls (L1) und des von dem zweiten Kernteil (21B) abgestrahlten Beleuchtungslichtstrahls (L2).

## Revendications

1. Dispositif (1) d'endoscope à balayage qui obtient une image parallaxe, comprenant :
une première partie de coeur (21a) qui rayonne un faisceau de lumière d'éclairage (L1) pour un premier point de vue vers un sujet (A), le faisceau de lumière d'éclairage (L1) comportant une première caractéristique optique ;
une deuxième partie de coeur (21b) qui est prévue parallèle à la première partie de coeur (21a) et qui rayonne un faisceau de lumière d'éclairage (L2) pour un deuxième point de vue vers le sujet (A) simultanément avec le rayonnement de la première partie de coeur (21a), le faisceau de lumière d'éclairage (L2) comportant une deuxième caractéristique optique différente de la première caractéristique optique ;
une unité d'entraînement (4, 7) qui est prévue en commun avec la première partie de coeur (21a) et la deuxième partie de coeur (21b) et qui balaye en deux dimensions le faisceau de lumière d'éclairage (L1) rayonné depuis la première partie de coeur (21a) et le faisceau de lumière d'éclairage (L2) rayonné depuis la deuxième partie de coeur (21b) en provoquant des vibrations des parties d'extrémité distale de la première partie de coeur (21a) et de la deuxième partie de coeur (21b) conjointement ;
une unité de réception de lumière (31) qui reçoit des faisceaux de lumière de retour, renvoyés depuis le sujet (A), du faisceau de lumière d'éclairage (L1) rayonné depuis la première partie de coeur (21a) et du deuxième faisceau de lumière d'éclairage (L2) rayonné depuis la deuxième partie de coeur (21b) ;
une unité de séparation de lumière (81) qui sépare les faisceaux de lumière de retour reçus par l'unité de réception de lumière (31) en un faisceau de lumière de retour possédant la première caractéristique optique et un faisceau de lumière de retour possédant la deuxième caractéristique optique ;
une première unité de détection de lumière (82a) qui convertit de manière photoélectrique le faisceau de lumière de retour séparé par l'unité de séparation de lumière (81) et possédant la première caractéristique optique pour délivrer en sortie un premier signal d'image capturée pour le premier point de vue ;
une deuxième unité de détection de lumière (82b) qui convertit de manière photoélectrique le faisceau de lumière de retour séparé par l'unité de séparation de lumière (81) et possédant la deuxième caractéristique optique pour délivrer en sortie un deuxième signal d'image capturée pour le deuxième point de vue ; et
une unité de génération d'image (9) qui génère une première image pour le premier point de vue sur la base du premier signal d'image capturée délivré en sortie depuis la première unité de détection de lumière (81a) et qui génère une deuxième image pour le deuxième point de vue sur la base du deuxième signal d'image capturée délivré en sortie depuis la deuxième unité de détection de lumière (81b).

2. Dispositif (1) d'endoscope à balayage selon la revendication 1,
dans lequel la première caractéristique optique est une première plage de longueur d'onde, et la deuxième caractéristique optique est une deuxième plage de longueur d'onde, qui diffère de la première plage de longueur d'onde.

3. Dispositif (1) d'endoscope à balayage selon la revendication 1,
dans lequel la première caractéristique optique est une première direction de polarisation, et la deuxième caractéristique optique est une deuxième direction de polarisation, qui diffère de la première direction de polarisation.

4. Dispositif (1) d'endoscope à balayage selon la revendication 1, comprenant un contrôleur (10) de source de lumière qui commande le faisceau de lumière d'éclairage (L1) rayonné depuis la première partie de coeur (21a) et possédant la première caractéristique optique et le faisceau de lumière d'éclairage (L2) rayonné depuis la deuxième partie de coeur (21b) et possédant la deuxième caractéristique optique de sorte que le sujet (A) est irradié simultanément.

5. Dispositif (1) d'endoscope à balayage selon la revendication 1, comprenant un contrôleur (10) de source de lumière qui commande le faisceau de lumière d'éclairage (L1) rayonné depuis la première partie de coeur (21a) et possédant la première caractéristique optique et le faisceau de lumière d'éclairage (L2) rayonné depuis la deuxième partie de coeur (21b) et possédant la deuxième caractéristique optique de sorte que le sujet (A) est irradié selon un mode de multiplexage à répartition dans le temps.

6. Dispositif (1) d'endoscope à balayage selon la revendication 1, comprenant un composant optique (12, 13) qui est prévu sur le côté d'extrémité distale de la première partie de coeur (21a) et de la deuxième partie de coeur (21b) pour provoquer la condensation des faisceaux de lumière d'éclairage (L1, L2).

7. Dispositif (1) d'endoscope à balayage selon la revendication 1, comprenant une unité de commande (10) qui synchronise l'unité d'entraînement (4, 7) et l'unité de génération d'image (9) l'une avec l'autre de sorte que des images des faisceaux de lumière de retour sont formées conformément aux vibrations de la première partie de coeur (21a) et de la deuxième partie de coeur (21b) provoquées par l'unité d'entraînement (4, 7).

8. Dispositif (1) d'endoscope à balayage selon la revendication 1, dans lequel l'unité d'entraînement (4, 7) fait vibrer les parties d'extrémité distale de la première partie de coeur (21a) et de la deuxième partie de coeur (21b) conjointement d'une manière telle que les trajectoires de balayage (S1, S2) du faisceau de lumière d'éclairage (L1) rayonné depuis la première partie de coeur (21a) et du faisceau de lumière d'éclairage (L2) rayonné depuis la deuxième partie de coeur (21b) sont déplacées l'une l'autre d'une distance prédéterminée (d) et présentent la même forme l'une l'autre.

9. Dispositif (1) d'endoscope à balayage selon la revendication 8, dans lequel l'unité de réception de lumière (31) reçoit les faisceaux de lumière de retour au niveau d'une position prédéterminée, et
dans lequel l'unité de génération d'image (9) génère des images dont les points de vue sont décalés l'un l'autre en parallèle d'une quantité correspondant à une distance prédéterminée en tant que première et deuxième images, sur la base d'informations incluant des positions de balayage du faisceau de lumière d'éclairage (L1) rayonné depuis la première partie de coeur (21a) et du faisceau de lumière d'éclairage (L2) rayonné depuis la deuxième partie de coeur (21b).
